# EUROPÄISCHE PATENTSCHRIFT

(11) **EP 0 901 353 B1**
(45) Veröffentlichungstag und Bekanntmachung des Hinweises auf die Patenterteilung: **11.07.2001**
(21) Anmeldenummer: 97920561.4
(22) Anmeldetag: 27.03.1997
(51) Int. Cl.: A61F 2/06

(54) **STENT**
STENT
EXTENSEUR

(30) Priorität: 29.03.1996 DE 19612615; 26.02.1997 DE 19707642
(43) Veröffentlichungstag der Anmeldung: 17.03.1999
(73) Patentinhaber: WILLY RÜSCH AG, D-71394 Kernen (DE)
(72) Erfinder: BRAUN, Michael, D-71522 Backnang (DE); SINGVOGEL, Armin, D-71686 Remseck (DE); KLEPETKO, Walter, A-1180 Wien (AT); BOLLIGER, Christoph, CH-4104 Oberwil (CH); FREITAG, Lutz, D-58675 Hemer (DE)
(74) Vertreter: KOHLER SCHMID + PARTNER
(86) Internationale Anmeldenummer: DE9700649
(87) Internationale Veröffentlichungsnummer: WO9736556

(56) Entgegenhaltungen:
- EP-A- 0 579 523
- EP-A- 0 621 016
- EP-A- 0 622 059
- EP-A- 0 679 372
- EP-A- 0 696 446
- EP-A- 0 696 447
- WO-A-92/06734
- WO-A-95/05132
- WO-A-95/17859
- WO-A-96/00103
- DE-A- 4 022 956
- DE-B- 1 766 921

## Beschreibung

Die Erfindung betrifft einen Stent zur Schienung und/oder zum Offenhalten eines Hohlraums, insbesondere eines Hohlorgans, wie dieser Stent im Oberbegriff des Anspruchs 1 definiert wird. Ein solcher Stent ist z.B. aus der Druckschrift WO 95/05132 bekannt.

In der DE 1766921 ist eine weitere Stentanordnung zur Schienung bzw. zum Offenhalten eines Hohlorgans beschrieben. Die bekannte Anordnung weist ein aus Filamenten bestehendes tubuläres und selbstexpandierendes Netz auf, das aus einer Vielzahl von ineinander geflochtenen Filamenten besteht. Gemäß einem Ausführungsbeispiel der DE 1766921 ist der Stent als armierter Schlauch ausgebildet, wobei das Netz innerhalb des Schlauchs fest eingebettet ist. Falls der Schlauch aus einem gewebeschonenden Material, beispielsweise aus einem Kunststoff, hergestellt ist, ergibt sich eine Stenteinrichtung die zur Schienung bzw. zum Offenhalten eines Hohlorgans geeignet ist. Da das Netz vollständig innerhalb der Schlauchwandung eingebettet ist, weist die Außenoberfläche des Schlauchs eine glatte Oberfläche auf, die zwar gewebeschonend sein kann, falls der Schlauch aus einem gewebeschonenden Material hergestellt ist, die sich aber möglicherweise gegenüber einer vorgegebenen plazierten Stellung innerhalb des Hohlorgans verschieben kann.

Demgegenüber ist es Aufgabe der vorliegenden Erfindung, einen Stent der obengenannten Art vorzustellen, der kostengünstig herstellbar ist, der eine sichere, gewebeschonende Plazierung innerhalb eines Hohlorgans oder Hohlkörpers ermöglicht und der eine positionsstabile Plazierung dauerhaft ermöglicht.

Diese Aufgabe wird gelöst durch einen Stent mit den Merkmalen des Anspruchs 1.

Das Ausbilden des erfindungsgemäßen Stents als einen Verbund zwischen drei Komponenten mit einem inneren Schlauch, einem darauf dicht anliegenden angeordneten tubulären Geflecht und einer auf der Außenoberfläche des Schlauchs angeordneten Beschichtung gewährleistet einen Stent, der nicht nur kostengünstig herzustellen ist, sondern der auch eine schonende und sichere Plazierung innerhalb beispielsweise eines Hohlorgans ermöglicht. Die äußere Beschichtung dient dazu, das Geflecht auf der Außenoberfläche des Schlauchs zu fixieren, wobei die Rückstellkräfte (Expansion des Stents aus einem unter Vorspannung stehendem gelängtem Stent kleineren Durchmessers in einen Zustand des Stents mit einem größeren Durchmesser und damit einhergehenden größeren Lumen) durch ein Zusammenwirken der elastischen Eigenschaften des Schlauchs im Verbund mit dem Geflecht bestimmt werden. Die Beschichtung ist derart ausgebildet, daß sich eine strukturierte Oberfläche des Stents ergibt, die durch die Struktur des Geflechts geprägt wird. Auf diese Weise, im Gegensatz zu dem herkömmlichen Stent, weist der erfindungsgemäße Stent eine strukturierte Oberfläche auf, die einen sicheren Halt innerhalb des Hohlorgans gewährleistet. Durch die Auswahl des Materials des Stents, insbesondere des Materials des Geflechts, können die Rückstellkräfte des Stents eingestellt werden. Auf diese Weise wird demzufolge ein kostengünstiger Stent geschaffen, der trotzdem eine sichere Platzierung innerhalb eines Hohlorgans gewährleistet. Die Beschichtung wird ausreichend dünn genug gehalten, damit die Struktur der Filamente zu einer in einzelnen Flächenabschnitten gewölbten Oberfläche des Stent führt. Diese strukturierte Oberfläche dient dazu, daß der Stent innerhalb eines Hohlorgans verbessert gesichert und dauerhaft positionsstabil gehalten werden kann.

Weitere Vorteile ergeben sich, wenn das Geflecht selbstexpandierbar ist. Auf diese Weise sind die Rückstellkräfte des Stents auch durch die Auswahl eines geeigneten Geflechts einstellbar und ergeben sich nicht nur aus den Materialeigenschaften des Schlauchs.

In einer bevorzugten Ausgestaltung der Erfindung haben die Filamente einen runden Querschnitt. Diese Maßnahme hat den Vorteil, daß herkömmliches Drahtmaterial als geflechtbildende Filamente verwendet werden kann. Zudem ist die einfache Strukturierung durch ein Filament mit einem runden Querschnitt ausreichend, um dem Stent eine geprägte Oberflächenstruktur zu verleihen.

Es ist vorteilhaft, wenn die Filamente Polyester, Kevlar (ein eingetragenes Warenzeichen), Glasfasern, und/oder Metall beinhalten oder aus diesen Materialien gefertigt sind. Polyester hat: den Vorteil, daß es als an sich bekanntes Material mit guten Verarbeitungseigenschaften zur Verfügung steht und dazu geeignet ist, einen stabilen Verbund mit dem Schlauch und der Beschichtung einzugehen. Durch die Auswahl des Materials eines Filaments, beispielsweise Polyester oder Kevlar (ein eingetragenes Warenzeichen), ist es möglich, die Rückstellkräfte des Stents zu beeinflussen. Kevlar (ein eingetragenes Warenzeichen) beispielsweise hat steifere Materialeigenschaften als Polyester und dient dazu, einen Stent zu schaffen, der stärkere Rückstellkräfte aufweist. Die Möglichkeit, die Rückstellkräfte einzustellen, wird durch die Verwendung von Glasfasern erweitert. Metallfilamente haben nicht nur die Eigenschaft, daß sie besonders stark armierte Stentstrukturen gewährleisten, sondern auch, daß die Plazierung und Lage des Stents anhand von Röntgenstrahlenuntersuchungen festgestellt werden kann.

In einer Weiterbildung dieses Ausführungsbeispiels beinhalten die Filamente ein Gemisch aus Glasfasern und Metall. Diese Maßnahme hat den Vorteil, daß die Rückstellkräfte eingestellt werden können, und daß gleichzeitig die Plazierung des Stents durch Röntgenstrahlung kontrolliert werden kann.

Im allgemeinen ist es vorteilhaft, wenn die Filamente röntgenstrahlendicht ausgebildet sind. Dies hat den Vorteil, daß unabhängig davon, ob die Rückstellkräfte durch beispielsweise Polyester, Kevlar (ein eingetragenes Warenzeichen) oder Metall gegeben sind, es trotzdem möglich ist, die Plazierung des Stents durch Röntgenstrahlen zu kontrollieren.

In einer vorteilhaften Weiterbildung dieses Ausführungsbeispiels ist Metallpulver oder sind Metallteile, beispielsweise Wolfram, im Schlauch oder in der Beschichtung enthalten. Diese Maßnahme hat den Vorteil, daß der Stent anhand von Röntgenstrahlenuntersuchungen nicht nur dadurch "sichtbar" wird, die Filamente sind beispielsweise aus einem Metall hergestellt, sondern daß auch anhand der im Stent eingebetteten Metallteile eine verbesserte Röntgenstrahlendichtheit erzielt wird. Auf diese Weise kann durch die Auswahl des Filamentmaterials die Rückstellkraft des Stents in einen expandierten Endzustand beeinflusst werden, und unabhängig davon ist die Plazierung des Stents mittels Röntgenstrahlung kontrolliert möglich.

Es ist besonders vorteilhaft, wenn der Schlauch und/oder die Beschichtung Silikon beinhaltet bzw. beinhalten. Dies hat den Vorteil, daß Silikon ein besonders einfach zu verarbeitendes Material ist, das zusätzlich gute gewebeverträgliche Eigenschaften aufweist.

Es ist vorteilhaft, wenn die Filamente fest fixierte, geschützte Enden aufweisen. Dies hat den Vorteil, daß die Filamentenden nicht die Beschädigung des Gewebes eines Hohlorgans auslösen können, und daß die strukturelle Stabilität des Stents in den Endbereichen definiert verbessert wird. Das Geflecht kann sich im Endbereich nicht schirmartig nach außen wölben.

In einer Weiterbildung dieses Ausführungsbeispiels sind benachbarte Filamentenden paarweise miteinander verbunden. Dies hat den Vorteil, daß die Filamentenden gefangen sind und sich nicht frei ausspreizen können.

In einer bestimmten Form dieser Weiterbildung werden die benachbarten Filamentenden durch eine Überzugskappe miteinander verbunden. Diese Überzugskappe kann schlauch- oder kuvertartig gestaltet werden. Dies hat den Vorteil, daß eine relativ einfache mechanische Konstruktion, nämlich die Überzugskappe, zu einem zuverlässigen Schutz und zur Lokalisierung der Filamentenden führt.

In einer anderen Form dieser Weiterbildung sind die benachbarten Filamentenden jeweils in einem gemeinsamen Filamentenschlauch gefangen. Der Schlauch schützt die Filamentenden und hält die Enden von mindestens zwei Filamenten zusammen.

Es ist aber auch möglich, die Filamentenden zusammenzuschweißen, beispielsweise durch Ultraschallverschweißung. Diese Maßnahme hat den Vorteil, daß kein zusätzliches Material benötigt wird, um eine sichere Lokalisierung und einen sicheren Schutz der Filamentenden zu gewährleisten.

Es ist weiterhin möglich, die Filamentenden fest auf den Silikonschlauch zu kleben und mit einem Silikonüberzug (Film) zu sichern. Die Kreuzungspunkte an den Enden können mittels beispielsweise eines Seidenfadens verknotet bzw. umwickelt werden. Die Kombination des Innenschlauchs und der Überzugskappe bietet den Vorteil, daß sich die Enden beim Strecken des Stents nicht auflösen. Es ist auch möglich, zur besseren Erkennung der Stentenden, wenn der Stent im Körper liegt, die Enden farblich zu markieren. Die Innenoberfläche des Stents kann derart strukturiert sein, daß eine gute und dauerhafte Benetzung von Fluiden und/oder Zellgewebe möglich ist.

Bevorzugt weist der Schlauch eine über die Filamentenden ausgebildete Umstülpung auf. Dies hat den Vorteil, daß der Schlauch und die Filamentenden in Kombination zu gefangenen Filamentenden führen, ohne daß zusätzliche Materialverbindungen hergestellt werden müssen.

In bevorzugter Ausgestaltung weist der Stent über die geprägte strukturierte Außenoberfläche vorstehende Erhebungen auf.

Dies hat den Vorteil, daß der Stent neben der strukturierten Außenoberfläche über Mittel verfügt, die eine gewünschte ortsfeste Endlage eines Stents im Hohlorgan zusätzlich sichern. Die Erhebungen können beliebig auf der Außenumfangsoberfläche des Stents verteilt sein und haben die Wirkung, daß sie sich im angrenzenden Gewebe verteilen, verhaken und/ oder in dieses eindrücken und dadurch die Lage eines Stents zusätzlich sichern.

Die Erhebungen sind in einer weiteren Ausgestaltung der Erfindung durch freie Enden von Filamentstücken gebildet, die zumindest abschnittsweise neben bzw. parallel zu den das Geflecht bildenden Filamenten verlaufen.

Dies hat den Vorteil, daß zur Erstellung derartiger Erhebungen nur ein zweiter Filamentfaden benötigt wird, der beispielsweise parallel zu den das Geflecht bildenden Filamentfäden verlaufen kann. Dieser zweite Filamentfaden, der die Geflechtstruktur bzw. die Geflechtstabilität nicht beeinflussen muß, wird nach gewünschten Abschnittslängen durchgetrennt und die dadurch entstehenden freien Enden werden über die Außenoberfläche des Stents vorstehend nach außen gebogen, so daß eine Außenoberfläche mit zahlreichen Haken entsteht, die sich in einem angrenzenden Gewebe verkrallen können.

Bei einer weiteren Ausgestaltung des erfindungsgemäßen Stents sind die Erhebungen durch Anker gebildet, die im gelängten Zustand des Stents auf der Außenoberfläche des Stents flach anliegen und mit einem ersten Ende an der Außenoberfläche des Stents ortsfest befestigt sind und mit einem freien zweiten Ende im expandierten Zustand des Stents von der Außenoberfläche des Stents beabstandet sind.

Dies hat den Vorteil, daß derartige Anker auch nachträglich auf einer strukturierten Außenoberfläche eines Stents angebracht werden können. Die Anker entfalten sich erst bei der Expansion des Stents und dringen dabei in das angrenzende Gewebe ein. Im gelängten Zustand eines Stents liegen diese Anker flach auf der Außenoberfläche eines Stents an, so daß die Plazierung eines Stents durch diese Anker nicht erschwert wird.

In einer weiteren Ausführungsform eines erfindungsgemäßen Stents sind die das Geflecht bildenden Filamente unterschiedlich weit voneinander beabstandet und/oder die Filamente weisen einen unterschiedlichen Durchmesser auf.

Über die Variation von Geflechtsarten und/oder Maschenweiten sowie über die Dicken der einzelnen Filamentfäden lassen sich vorbestimmbare Rückstellkräfte in einem Stent verwirklichen, so daß je nach Größe und Beschaffenheit einer Hohlorganengstelle immer der dafür bestmöglich geeignete Stent ausgewählt werden kann, damit dieser Stent eine gewünschte Lumenerweiterung im entsprechenden Hohlorgan dauerhaft sichert und eine unbeabsichtigte Migration des Stents ausgeschlossen werden kann.

Ist das Geflecht eines Stents bei einer weiteren Ausführungsform über einer in axialer und/oder radialer Richtung unterschiedliche Kontur aufweisenden Form hergestellt, so kann noch zusätzlich in Abhängigkeit vom axialen Verlauf eines Stents die Rückstellkraft innerhalb eines Stents variiert werden. Unterschiedliche Formen eines Geflechts lassen sich auch über eine thermische Verformung herstellen.

Es ist besonders vorteilhaft, wenn der Stent derart ausgebildet ist, daß er mittels einer Applikationseinrichtung in einen Hohlkörper einbringbar ist. Dies hat den Vorteil, daß die Plazierung des Stents erleichtert wird und reproduzierbare Methoden zur Plazierung des erfindungsgemäßen Stents erarbeitet werden können.

In einer Weiterbildung dieses Ausführungsbeispiels weist die Applikationseinrichtung eine Fang- und Schiebeeinrichtung, eine Applikationshülse und einen Kegel-Pfropfen auf. Dies hat den Vorteil, daß der Stent mit dieser Applikationseinrichtung nicht nur in ein Hohlorgan eingebracht, sondern auch mit dieser Einrichtung plaziert werden kann. Der Kegel-Pfropfen dient zur leichteren Handhabung in der Applikationshülse.

In einer Weiterbildung dieser Ausführungsform weist die Fang- und Schiebeeinrichtung einen aufgespreizten Fängerkorb aus einem Kunststoff- oder Metallgeflecht auf. Dies hat den Vorteil, daß die Fang- und Schiebeeinrichtung in der Lage ist, den Stent sicher zu fangen, und, nachdem der Stent innerhalb der Applikationshülse zusammengelegt gehalten ist, wieder freizugeben. Die Fang- und Schiebeeinrichtung ist derart ausgebildet und dimensioniert, daß sie in das Lumen der Applikationshülse einschiebbar ist.

Der Fängerkorb weist gegenüber der Applikationshülse und/ oder dem Stent eine erhöhte Gleitfähigkeit auf, wenn in das Geflecht des Fängerkorbs eine Silikonmasse eingearbeitet ist (reibungsmindernd sind beispielsweise auch Beschichtungen aus Teflon (ein eingetragenes Warenzeichen) oder polyäthylen). Dies gewährleistet ein sicheres und leichteres Entfernen der Fang- und Schiebeeinrichtung vom darin eingefangenen Stent.

Vorteilhaft ist es, wenn die Fang- und Schiebeeinrichtung ein Lumen aufweist. Dies hat den Vorteil, daß das Lumen der Fang- und Schiebeeinrichtung dazu geeignet ist, über optische Mittel eine Beobachtung der Plazierung des Stents innerhalb eines Hohlorgans zu gewährleisten.

In einer vorteilhaften Weiterbildung ist die Fang- und Schiebeeinrichtung eine Unterstützung für den Stent, wenn ein Bereich des freien Endes der Schiebevorrichtung in das Stentlumen hineinragt. Kleine Stentgrößen < 10 mm Innendurchmesser, können beim Herausschieben aus der Applikationshülse nicht beschädigt werden, weil sie aus dem Innenbereich heraus gestützt werden. Ein zusätzlicher Einsatz von Gleitmittel im Außenbereich des Stents ist möglich, damit die Reibung zwischen der Innenoberfläche der Applikationshülse und der Außenoberfläche des Stents herabgesetzt wird.

Um den Stent aus dem Fängerkorb zu entfernen, ist es notwendig, diesen nicht ganz in die Applikationshülse zu ziehen. Mit einem Kegel-Pfropfen wird der Stent in der Applikationshülse fixiert und der Fängerkorb herausgezogen. Das überstehende Geflecht kann dann in die Applikationshülse, z.B. unter Zuhilfenahme der Kegelpfropfen-Rückseite schonend eingeführt werden. Wird der Kegel-Pfropfen nicht benützt, kann dies zu Deformationen und Beschädigungen am Stent, besonders bei den Größen kleiner als 10 mm Innendurchmesser führen.

Die Kombination von Fangeinrichtung und Applikationshülse kann so gestaltet werden, daß beide Gegenstände mit geringen Wandstärken hergestellt werden können. Die Applikationshülse weist dabei eine Wandstärke auf, die so dünn ist, daß sie durch die Fangeinrichtung stabilisiert werden muß.

Durch die Materialauswahl ist es möglich, ein hohes Maß an Flexibilität zu erreichen. Dies hat den Vorteil, daß eine sichere, einfache und körperschonende Implantation des Stents gewährleistet wird.

Ein Verfahren zum Einbringen des Stents kann vorsehen, daß der Stent mittels des sich konisch ausspreizenden Endes der Fang- und Schiebeeinrichtung in das Lumen der Applikationshülse eingezogen wird, der Stent mittels des Kegel-Pfropfens innerhalb des Lumens der Applikationshülse fixiert wird, die Fang- und Schiebeeinrichtung von dem Stent getrennt und aus dem Lumen der Applikationshülse herausgezogen wird, der Kegel-Pfropfen von der Applikationshülse getrennt wird, die Fang- und Schiebeeinrichtung umgedreht und wieder in das Lumen der Applikationshülse eingeschoben wird, optische Mittel durch das Lumen der Fang- und Schiebeeinrichtung einschiebbar sind, die Applikationshülse in den Hohlkörper eingebracht wird, und der Stent mittels der Fang- und Schiebeeinrichtung aus der Applikationshülse heraus in den Hohlkörper plaziert wird. Dies hat den Vorteil, daß der Stent auf eine einfache und sichere Art und Weise mittels der Applikationseinrichtung zunächst in der Applikationshülse gefangen und kollabiert gehalten werden kann. Die Fang- und Schiebeeinrichtung wird nicht nur dazu benutzt, um den Stent in die Applikationshülse einzuziehen, sondern auch um den Stent aus der Applikationshülse in das Hohlorgan herauszuschieben. Die Fang- und Schiebeeinrichtung weist demzufolge ein Ende mit einer Fangeinrichtung und ein der Fangeinrichtung gegenüberliegendes Ende auf, das zum Schieben des Stents aus der Applikationshülse heraus in das Hohlorgan ausgebildet ist.

Ein Verfahren zur Herstellung eines Stents kann folgende Schritte vorsehen: das Geflecht wird auf die Außenoberfläche des Schlauchs dicht anliegend aufgebracht, und danach werden der Schlauch und das Geflecht in eine Flüssigkeit, beispielsweise Silikon, getaucht. Diese Flüssigkeit verfestigt sich und bildet die zweite Beschichtung.

Die freien Enden des Geflechts können auch jeweils miteinander verschweißt werden und zusätzlich durch eine Klebeschicht geschützt werden. Enthält die Klebeschicht Bariumsulfat, so sind die Stentenden röntgenschattengebend.

Dies hat den Vorteil, daß der erfindungsgemäße Stent einfachst hergestellt werden kann.

Weitere Vorteile der Erfindung ergeben sich aus der Beschreibung und der Zeichnung.

Es zeigt:
- Fig. 1a: einen dem Stent zugrunde liegenden Schlauch;
- Fig. 1b: ein dem Stent zugrunde liegendes Geflecht;
- Fig. 1c: eine dem erfindungsgemäßen Stent zugrunde liegende Beschichtung;
- Fig. 1d: eine schematische Darstellung des den erfindungsgemäßen Stent bildenden Verbunds zwischen Schlauch, Geflecht und Beschichtung;
- Fig. 2: einen schematischen Querschnitt bzw. eine Endansicht des erfindungsgemäßen Stents gemäß Fig. ld;
- Fig. 3a: ein erfindungsgemäßes Ausführungsbeispiel von gefangenen freien Filamentenden mittels einer Überzugskappe;
- Fig. 3b: ein weiteres erfindungsgemäßes Ausführungsbeispiel von gefangenen Filamentenden mittels eines Filamentenschlauchs;
- Fig. 3c: ein Ausführungsbeispiel mit verschweißten Filamentenden;
- Fig. 3d: eine Umstülpung des Schlauchs, das den Zusammenhalt der Filamentenden bewirkt;
- Fig. 4a: eine erfindungsgemäße Applikationseinrichtung zur Plazierung des erfindungsgemäßen Stents;
- Fig. 4b: eine Applikationseinrichtung gemäß Fig. 4a mit eingezogenem Stent und Kegel-Pfropfen;
- Fig. 4c: eine Applikationseinrichtung mit einer gegenüber Fig. 4a gedrehten Fang- und Schiebeeinrichtung;
- Fig. 5: einen Stent mit Erhebungen auf der Außenumfangsfläche, die durch freie Enden von Filamentstücken gebildet sind;
- Fig. 6: einen Stent mit auf der Außenoberfläche angebrachten Ankern im gelängten Zustand;
- Fig. 7: einen Stent gemäß Fig. 6 im expandierten Zustand;
- Fig. 8: einen Stent mit in Abhängigkeit vom axialen und radialen Verlauf unterschiedlicher Oberflächenkontur;
- Fig. 9: einen Stent im expandierten Zustand mit jeweils erweitertem Lumen im freien Endbereich.

Die Figuren sind teilweise sehr schematisch dargestellt, um die wesentlichen erfinderischen Merkmale zu verdeutlichen. In den Darstellungen sind die Dimensionen nur beispielhaft und nicht maßstäblich zu verstehen.

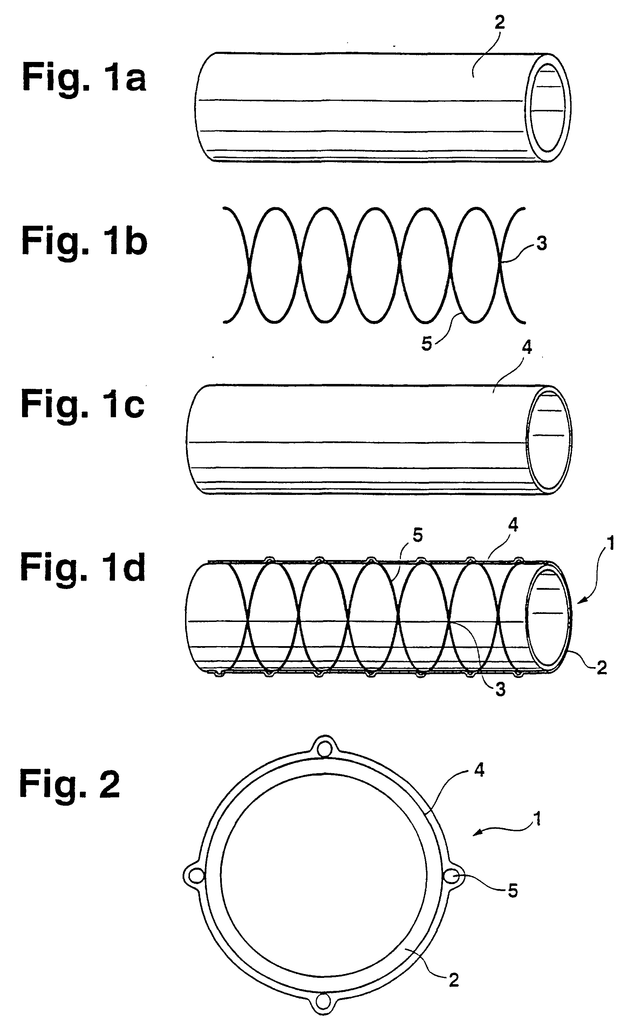

Fign. la-ld veranschaulichen den Aufbau des erfindungsgemäßen Stents 1. Der Stent 1 besteht aus - wie in Fig. la dargestellt ist - einem Schlauch 2 aus einem elastischen Material, einem an der Oberfläche des Schlauchs 2 dicht anliegenden tubulären Geflecht 3 - wie in Fig. 1 b gezeigt - das aus einer Vielzahl von Filamenten 5 zusammengeflochten ist, sowie einer auf der Außenoberfläche des Schlauchs 2 aufgetragenen Beschichtung 4 (Fig. 1c). Fig. ld zeigt den Stent 1 in zusammengebautem Zustand. Der innere Schlauch 2 weist eine glatte innere Oberfläche auf und ist durch den Verbund mit dem aus Filamenten 5 bestehenden Geflecht 3 armiert, wobei der Verbund zwischen dem tubulären Geflecht 3 und dem Innenschlauch 2 mittels der Beschichtung 4 geschaffen wird. Gemäß Fig. 1d ist die Beschichtung 4 derart ausgebildet, daß die mittels der Filamente 5 auf der Außenoberfläche des Schlauchs 2 ausgeprägte Struktur durch die Beschichtung 4 hindurchragt und zu einer strukturierten Außenoberfläche der Beschichtung 4 führt.

Fig. 2 zeigt einen schematischen Querschnitt bzw. eine Endansicht des erfindungsgemäßen Stents. Der innere Schlauch 2 sowie das aus Filamenten 5 bestehende Geflecht 3, das auf der Außenoberfläche des inneren Schlauchs 2 dicht anliegt, sind mittels der relativ dünnen Beschichtung 4 haftend zusammengehalten. In dem Ausführungsbeispiel gemäß Fig. 2 weist der Stent 1 eine strukturierte Außenoberfläche auf, die durch die mit rundem Querschnitt versehenen Filamente 5 auf der Außenoberfläche des Schlauchs 2 ausgeprägt ist. Dies wird dadurch erreicht, daß die Beschichtung 4 ausreichend dünn ist, um die Räume zwischen den Filamenten 5 nicht vollständig auszufüllen, wobei die durch die Filamente 5 geprägte Struktur der Außenoberfläche des Schlauchs 2 einfach mittels der Beschichtung 4 dichtend überzogen ist.

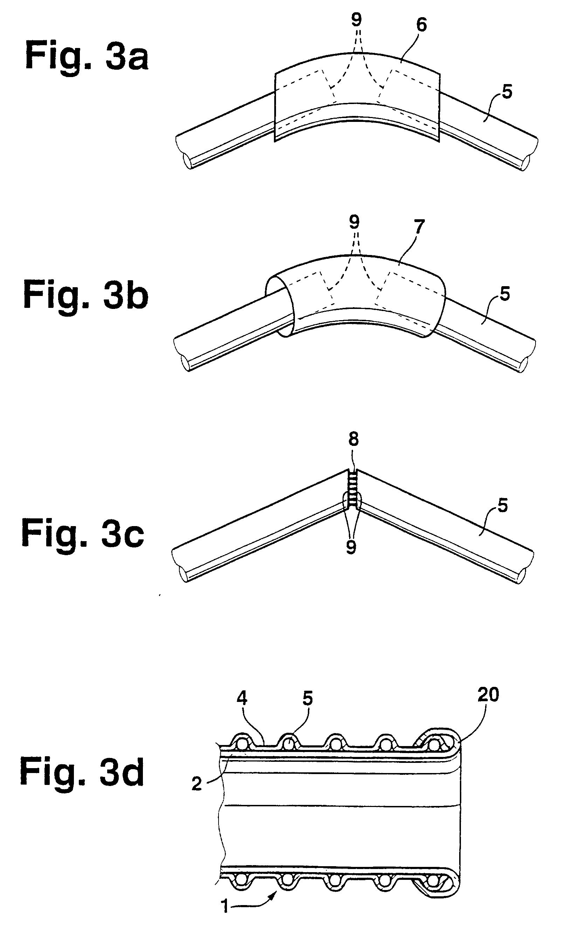

Die Ausführungsbeispiele gemäß Fign. 3a-3d zeigen verschiedene Möglichkeiten, um Filamentenden 9 fest zusammenzuhalten. In Fig. 3a werden die Enden 9 des Filaments 5 durch eine Überzugskappe 6 miteinander geschützt verbunden. Gemäß Fig. 3b werden die Enden 9 der Filamente 5 jeweils durch einen gemeinsamen Filamentenschlauch 7 miteinander verbunden. Die freien Enden 9 der Filamente 5 sind gemäß Fig. 3c durch eine Verschweißung 8 zusammengefügt und gefangen. Gemäß Fig. 3d ist es alternativ möglich, die freien Enden der Filamente 5 durch eine Umstülpung 20 des inneren Schlauchs 2 einzufangen.

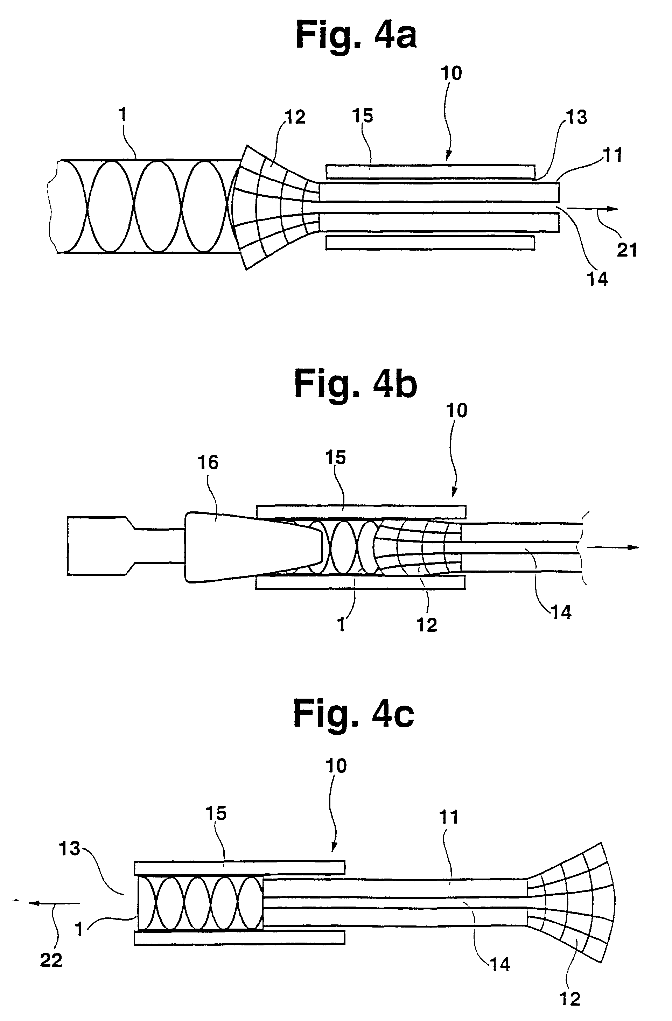

Fign. 4a und Fig. 4b zeigen eine Applikationseinrichtung 10, die dazu geeignet ist, den Stent 1 in einen Hohlkörper bzw. in ein Hohlorgan einzuführen. Die Applikationseinrichtung 10 gemäß Fign. 4a und 4b besteht aus einer äußeren Applikationshülse 15 sowie einer inneren Fang- und Schiebeeinrichtung 11. Die innere Fang- und Schiebeeinrichtung 11 weist an ihrem einen Ende eine ausgespreizte Fangeinrichtung 12 auf und ist auf dem der Fangeinrichtung 12 gegenüberliegenden Ende glatt ausgebildet. Zudem ist die Fang- und Schiebeeinrichtung 11 mit einem Lumen 14 versehen. In Fig. 4a wird der Stent 1 mittels des ausgespreizten Endes der Fangeinrichtung 12 in die Applikationshülse 15 der Applikationseinrichtung 10 in Richtung des Pfeils 21 hineingezogen. Dazu ist der Außendurchmesser der Fang- und Schiebeeinrichtung 11 derart dimensioniert, daß sie innerhalb eines Lumens 13 der Applikationshülse 15 schiebbar angeordnet werden kann. Gemäß Fig. 4c wird ein bereits eingefangener Stent 1 mittels der Fang- und Schiebeeinrichtung 11 in Richtung Pfeil 22 aus der Applikationshülse 15 hinausgeschoben und innerhalb eines Hohlorgans plaziert.

Um die Applikationseinrichtung gemäß Fign. 4a, 4b und 4c zu benutzen, wird zunächst der Stent 1 mittels des ausgespreizten Endes 12 der Fang- und Schiebeeinrichtung 11 in die Applikationshülse 15 gemäß Fig. 4a hineingezogen. Nachdem der Stent 1 sich gemäß Fig. 4b vollständig innerhalb der Applikationshülse 15 befindet, wird der Stent 1 mittels des Kegel-Pfropfens 16 innerhalb des Lumens 13 der Applikationshülse 15 fixiert. Eine gezielte Bewegung der Fangeinrichtung 12 in Pfeilrichtung 21 gibt nun den Stent 1 frei. Der Kegel-Pfropfen 16 wird von der Applikationshülse 15 getrennt und die Fang- und Schiebeeinrichtung 11 wird aus der Applikationshülse 15 herausgezogen, umgedreht und mit ihrem anderen Ende wieder in die Applikationshülse 15 gemäß Fig. 4c hineingeschoben. Eine optische Beobachtung des Plazierens des Stents 1 wird durch in ein Lumen 14 einführbare Instrumente ermöglicht. Die Applikationseinrichtung 10 wird danach innerhalb eines Hohlorgans oder Hohlkörpers plaziert, und durch eine Verschiebung der Fang- und Schiebeeinrichtung 11 wird der Stent 1 innerhalb des Hohlorgans plaziert.

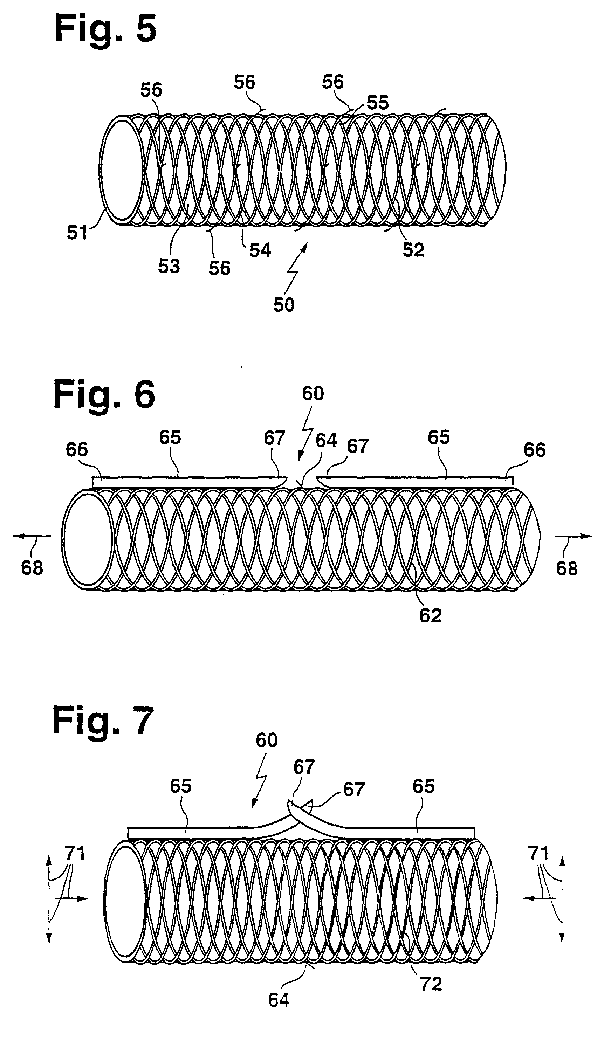

Fig. 5 zeigt einen Stent 50, der aus einem Schlauch 51, einem Geflecht 52 und einer Beschichtung 53 gebildet ist. Neben Filamentfäden 54, aus denen das Geflecht 52 hergestellt ist, verläuft neben den Fäden 54 ein zweiter Filamentfaden 55, der abschnittsweise unterbrochen ist, und dessen freie Enden 56 über das Geflecht 52 und die Beschichtung 53 vorstehen. Die freien Enden 56 bilden Widerhaken für ein an die Stentaußenoberfläche angrenzendes Gewebe.

Fig. 6 zeigt eine weitere Ausführungsform eines Stents 60 im gelängten Zustand, der aus einem Schlauch 61, einem Geflecht 62 und einer Beschichtung 63 aufgebaut ist. An einem Flächenabschnitt der Außenoberfläche 64 des Stents 60 sind Anker 65 aufgebracht. Die Anker 65 sind über ein jeweiliges erstes Ende 66 ortsfest mit der Außenoberfläche 64 des Stents 60 verbunden. Ein zweites Ende 67 schmiegt sich an die Außenoberfläche 64 des Stents 60 an. Der Stent 60 ist in Pfeilrichtungen 68 gelängt.

In Fig. 7 ist der Stent 60 der Fig. 6 im expandierten Zustand gezeigt. Der Stent 60 ist in Pfeilrichtungen 71 expandiert, so daß sich ein vergrößertes Lumen 72 ergibt. Während der Expansion richten sich die zweiten Enden 67 der Anker 65 auf, so daß sie von der Außenoberfläche 64 beabstandet sind. Über die zweiten Enden 67 können sich die Anker 65 des Stents 60 in einer angrenzenden Oberfläche verhaken bzw. verkrallen.

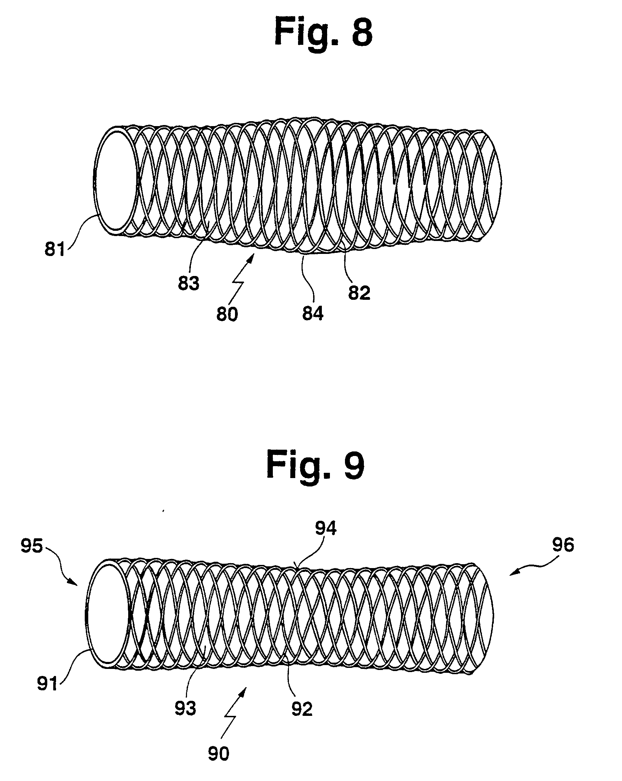

Fig. 8 zeigt einen Stent 80, der ebenfalls aus einem Schlauch 81, einem Geflecht 82 und einer Beschichtung 83 besteht. Die Form des Stents 80 ist in Abhängigkeit von seiner axialen und radialen Erstreckung unterschiedlich. Der Stent 80 weist eine bauchige Kontur 84 auf, die der Stent 80 im expandierten Zustand einnimmt. Die bauchige Kontur 84 wird dadurch erreicht, daß das Geflecht 82 über einer Form geflochten wird, die diese bauchige Kontur 84 aufweist. Die Kontur einer Form kann beliebig sein und ist auf den Anwendungsfall abzustimmen. Die in der Fig. 8 gezeigte bauchige Kontur 64 kann dauerhaft auch über eine thermische Verformung erreicht werden.

Fig. 9 zeigt eine weitere Ausführungsform eines Stents 90, der auf einem Schlauch 91 ein Geflecht 92 trägt, das von einer Beschichtung 93 überdeckt ist. Im Querschnitt weist die Außenkontur des Stents 90 über die gesamte axiale Erstrekkung des Stents 90 einen konkaven Verlauf 94 auf, so daß der Stent 90 freie Enden 95, 96 bildet, die jeweils ein vergrößertes Lumen im Endbereich des Stents 90 begrenzen. Die freien Enden 95, 96 können noch durch Ringstrukturen auf der Außenoberfläche des Stents 90 verstärkt werden. Diese Ringstrukturen können auch an beliebigen Abschnitten eines Stents unabhängig von dem Ausführungsbeispiel der Fig. 9 an der Außenoberfläche eines Stents angebracht werden.

## Patentansprüche

1. Stent zur Schienung und/oder zum Offenhalten eines Hohlorgans mit einer aus einem elastischen Material ausgebildeten ersten Beschichtung (2), einem an der Außenoberfläche der ersten Beschichtung (2) dicht anliegenden aus Filamenten (5) bestehenden tubulären Geflecht (3) und einer auf dem Geflecht (3) sowie auf der Außenoberfläche der ersten Beschichtung (2) aufgetragenen, das Geflecht (3) auf der Außenoberfläche der ersten Beschichtung (2) haltenden zweiten Beschichtung (4), wobei der Durchmesser der das Geflecht (3) bildenden Filamente (5) größer ist als die Dicke der zweiten Beschichtung (4), dadurch gekennzeichnet, daß die erste Beschichtung als Schlauch (2) mit einer glatten inneren Oberfläche ausgebildet ist, auf dem das tubuläre Geflecht (3) aufliegt, und daß die zweite Beschichtung als eine sich verfestigende Beschichtung (4) durch Eintauchen in eine Flüssigkeit auf den Schlauch (2) aufgetragen ist, die eine durch das Geflecht (3) geprägte strukturierte Außenoberfläche bildet.

2. Stent nach Anspruch 1, dadurch gekennzeichnet, daß das Geflecht (3) selbstexpandierbar ist.

3. Stent nach einem der vorhergehenden Ansprüche, dadurch gekennzeichnet, daß die Filamente (5) einen runden Querschnitt aufweisen.

4. Stent nach einem der vorhergehenden Ansprüche, dadurch gekennzeichnet, daß die Filamente (5) Polyester, Kevlar, Glasfasern und/oder Metall beinhalten oder aus diesen Materialien gefertigt sind.

5. Stent nach einem der vorhergehenden Ansprüche, dadurch gekennzeichnet, daß die Filamente (5) röntgenstrahlendicht ausgebildet sind.

6. Stent nach einem der Ansprüche 1 bis 5, dadurch gekennzeichnet, daß im Schlauch (2) oder in der Beschichtung (4) ein Metallpulver oder Metallteile, beispielsweise Wolfram, enthalten sind.

7. Stent nach einem der vorhergehenden Ansprüche, daß der Schlauch (2) und/oder die sich verfestigende Beschichtung (4) Silikon beinhalten oder aus diesem Material gefertigt sind.

8. Stent nach einem der vorhergehenden Ansprüche, dadurch gekennzeichnet, daß die Filamente (5) fest fixierte, insbesondere geschützte Enden (9) aufweisen.

9. Stent nach Anspruch 8, dadurch gekennzeichnet, daß benachbarte Filamentenden (9) paarweise miteinander verbunden sind.

10. Stent nach Anspruch 9, dadurch gekennzeichnet, daß die benachbarten Filamentenden (9) durch eine Überzugskappe (6) miteinander verbunden sind.

11. Stent nach Anspruch 9, dadurch gekennzeichnet, daß die benachbarten Filamentenden (9) in einem gemeinsamen Filamentenschlauch (7) gefangen sind.

12. Stent nach Anspruch 9, dadurch gekennzeichnet, daß die benachbarten Filamentenden (9) verschweißt (8) sind, beispielsweise durch Ultraschallverschweißung.

13. Stent nach einem der Ansprüche 1 bis 12, dadurch gekennzeichnet, daß der Schlauch (2) eine über die Enden der Filamente ausgebildete Umstülpung (20) aufweist.

14. Stent nach einem der Ansprüche 1 bis 13, dadurch gekennzeichnet, daß der Stent (50; 60; 80) über die geprägte strukturierte Außenoberfläche vorstehende Erhebungen (56; 65; 84; 95, 96) aufweist.

15. Stent nach Anspruch 14, dadurch gekennzeichnet, daß die Erhebungen durch freie Enden (56) von Filamentstücken gebildet sind, die zumindest abschnittsweise neben bzw. parallel zu den das Geflecht (52) bildenden Filamentfäden (54) verlaufen.

16. Stent nach Anspruch 14 oder 15, dadurch gekennzeichnet, daß die Erhebungen durch Anker (65) gebildet sind, die im gelängten Zustand des Stents (60) auf der Außenoberfläche (64) des Stents (60) flach anliegen und mit einem ersten Ende (66) an der Außenoberfläche (64) des Stents (60) ortsfest befestigt sind und mit einem freien zweiten Ende (67) im expandierten Zustand des Stents (60) von der Außenoberfläche (64) des Stents (60) beabstandet sind.

17. Stent nach einem der Ansprüche 1 bis 16, dadurch gekennzeichnet, daß die das Geflecht (52; 62; 82; 92) bildenden Filamentfäden unterschiedlich weit voneinander beabstandet sind und/oder einen unterschiedlichen Durchmesser aufweisen.

18. Stent nach einem der Ansprüche 1 bis 17, dadurch gekennzeichnet, daß das Geflecht (82; 92) über einer in axialer und/oder radialer Richtung unterschiedliche Kontur aufweisenden Form hergestellt ist.

19. Stent nach einem der Ansprüche 1 bis 17, dadurch gekennzeichnet, daß die Form des Geflechts (82; 92) über eine thermische Verformung hergestellt ist.

20. Stent nach einem der vorhergehenden Ansprüche, dadurch gekennzeichnet, daß der Stent (1) derart ausgebildet ist, daß er mittels einer Applikationseinrichtung (10) in einen Hohlkörper einbringbar ist.

21. Stent nach Anspruch 20, dadurch gekennzeichnet, daß die Applikationseinrichtung (10) eine Fang- und Schiebeeinrichtung (11), eine Applikationshülse (15) und einen Kegel-Pfropfen (16) aufweist.

22. Stent nach Anspruch 21, dadurch gekennzeichnet, daß die Fang- und Schiebeeinrichtung (11) ein sich weitgehend konisch ausspreizendes Ende (12) aufweist.

23. Stent nach Anspruch 21 oder 22, dadurch gekennzeichnet, daß die Fang- und Schiebeeinrichtung (11) derart ausgebildet und dimensioniert ist, daß sie in das Lumen (13) der Applikationshülse (15) einschiebbar ist.

24. Stent nach einem der Ansprüche 21 bis 23, dadurch gekennzeichnet, daß die Fang- und Schiebeeinrichtung (11) ein Lumen (14) aufweist.

## Claims

1. Stent for the splinting and/or holding-open of a body cavity having
a first layer (2) made from an elastic material;
a tubular weave (3) seating firmly on the outer surface of the first layer (2) and made from filaments (5) and
a second layer (4) applied to both the weave (3) as well as the outer surface of the first layer (2) which holds the weave (3) on the outer surface of the first layer (2), wherein the diameter of the filaments (5) forming the weave (3) is larger than the thickness of the second layer (4), characterized in that the first layer is a tube (2), having a smooth inner surface, and on which the tubular weave (3) seats, and the second layer is fashioned on the tube (2) as self-solidifying through layer (4), self-solidification following dipping into a fluid to form a structured outer surface defined by the weave (3).

2. Stent according to claim 1, characterized in that the weave (3) is self-expanding.

3. Stent according to anyone of the preceding claims, characterized in that the filaments (5) have a rounded cross section.

4. Stent according to any one of the preceding claims, characterized in that the filaments (5) contain or are made from polyester, kevlar, fiber glass and/or metal.

5. Stent according to any one of the preceding claims, characterized in that the filaments (5) are impervious to X-rays.

6. Stent according to any one of the claims 1 through 5, characterized in that a metallic powder or metallic pieces are contained in the tube (2) or in the coating (4), e.g. tungsten.

7. Stent according to any one of the preceding claims, characterized in that the tube (2) and/or the coating (4) contain silicon or are made from same.

8. Stent according to any one of the preceding claims, characterized in that the filaments (5) have fixed ends (9) which are, in particular, protected.

9. Stent according to claim 8, characterized in that neighbouring filament ends (9) are pairwise connected to each other.

10. Stent according to claim 9, characterized in that the neighbouring filament ends (9) are connected to each other by means of a covering cap (6).

11. Stent according to claim 9, characterized in that the neighbouring filament ends (9) are captured in a common filament tube (7).

12. Stent according to claim 9, characterized in that the neighbouring filament ends (9) are welded (8) together, e.g. by means of ultrasound weldment.

13. Stent according to any one of the claims 1 through 12, characterized in that the tube (2) has a fold-over (20) covering the ends of the filaments.

14. Stent according to any one of the claims 1 through 13, characterized in that the stent (50;60;80) has raised portions (56;65;84;95,96) protruding beyond the embossed structured outer surface.

15. Stent according to claim 14, characterized in that the raised portions are formed by the free ends (56) of filament pieces which extend at least in sections proximate or parallel to the filament threads (54) forming the weave (52).

16. Stent according to claim 14 or 15, characterized in that the raised portions are formed by anchors (65) which seat flatly on the outer surface (64) of the stent (60) in the elongated state of the stent (60) and are firmly attached in a fixed spatial location at a first end (66) thereof to the outer surface (64) of the stent (60) and which are separated from the outer surface (64) of the stent (60) at a second free end (67) thereof in an expanded state of the stent (60).

17. Stent according to any one of the claims 1 through 16, characterized in that the filament threads forming the weave (52;62;82;92) have differing separations with respect to each other and/or have a differing diameter.

18. Stent according to any one of the claims 1 through 17, characterized in that the weave (82;92) can be produced on a mould having differing shapes in the axial and/or radial directions.

19. Stent according to any one of the claims 1 through 17, characterized in that the shape of the weave (82;92) is produced by means of thermal shaping techniques.

20. Stent according to any one of the preceding claims, characterized in that the stent (1) is configured in such a fashion that it can be introduced into a body cavity using an application device (10).

21. Stent according to claim 20, characterized in that the application device (10) has a capture and displacement device (11), an application bushing (15) and a conical plug (16).

22. Stent according to claim 21, characterized in that the capture and displacement device (11) has a substantially conically spread-out end (12).

23. Stent according to claim 21 or 22, characterized in that the capture and displacement device (11) is shaped and dimensioned in such a fashion that it can be displaced into the lumen (13) of the application bushing (15).

24. Stent according to any one of the claims 21 through 23, characterized in that the capture and displacement device (11) has a lumen (14).

## Revendications

1. Extenseur pour éclisser et/ou maintenir ouvert un organe creux avec un premier revêtement (2) constitué par un matériau élastique, un treillis tubulaire (3) constitué par des filaments (5) reposant étroitement sur la surface externe du premier revêtement (2) et un second revêtement (4) appliqué sur la surface externe du premier revêtement (2), maintenant le treillis (3) sur la surface externe du premier revêtement (2), le diamètre des filaments (5) formant le treillis (3) étant supérieur à l'épaisseur du second revêtement (4), caractérisé en ce que le premier revêtement est sous forme de tuyau souple (2) avec une surface interne lisse sur lequel repose le treillis tubulaire (3), et en ce que le second revêtement est appliqué sur le tuyau souple (2) par immersion dans un liquide sous forme d'un revêtement (4) qui se solidifie et qui forme une surface externe structurée gaufrée par le treillis (3).

2. Extenseur selon la revendication 1, caractérisé en ce que le treillis (3) est autoexpansible.

3. Extenseur selon l'une des revendications précédentes, caractérisé en ce que les filaments (5) comportent une section droite ronde.

4. Extenseur selon l'une des revendications précédentes, caractérisé en ce que les filaments (5) contiennent du polyester, du Kevlar, des fibres de verre et/ou un métal ou sont constitués par ces matériaux.

5. Extenseur selon l'une des revendications précédentes, caractérisé en ce que les filaments (5) sont opaques aux rayons X.

6. Extenseur selon l'une des revendications 1 à 5, caractérisé en ce qu'une poudre métallique ou des parties métalliques, par exemple du tungstène, sont contenues dans le tuyau souple (2) ou dans le revêtement (4).

7. Extenseur selon l'une des revendications précédentes, caractérisé en ce que le tuyau souple (2) et/ou le revêtement (4) qui se solidifie contiennent du silicone ou sont constitués par ce matériau.

8. Extenseur selon l'une des revendications précédentes, caractérisé en ce que les filaments (5) comportent des extrémités (9) fixées solidement, en particulier des extrémités (9) protégées.

9. Extenseur selon la revendication 8, caractérisé en ce que les extrémités de filaments (9) voisines sont reliées entre elles par paires.

10. Extenseur selon la revendication 9, caractérisé en ce que les extrémités de filaments voisines (9) sont reliées entre elles par un capuchon de recouvrement (6).

11. Extenseur selon la revendication 9, caractérisé en ce que les extrémités de filaments voisines (9) sont reçues dans un tuyau souple de filaments (7) commun.

12. Extenseur selon la revendication 9, caractérisé en ce que les extrémités de filaments voisines (9) sont soudées (8), par exemple par soudage à ultrasons.

13. Extenseur selon l'une des revendications 1 à 12, caractérisé en ce que le tuyau souple (2) comporte un retroussis (20) formé au-dessus des extrémités des filaments.

14. Extenseur selon l'une des revendications 1 à 13, caractérisé en ce que l'extenseur (50 ; 60 ; 80) comporte des protubérances (56 ; 65 ; 84 ; 95, 96) qui font saillie au-dessus de la surface externe structurée gaufrée.

15. Extenseur selon la revendication 14, caractérisé en ce que les protubérances sont formées par des extrémités libres (56) de parties de filaments qui s'étendent au moins par segments à côté de ou parallèlement aux fils de filaments (54) formant le treillis (52).

16. Extenseur selon la revendication 14 ou 15, caractérisé en ce que les protubérances sont formées par des ancres (65) qui, à l'état allongé de l'extenseur (60), reposent à plat sur la surface externe (64) de l'extenseur (60) et sont fixées de manière fixe en position par une première extrémité (66) sur la surface externe (64) de l'extenseur (60) et sont distantes de la surface externe (64) de l'extenseur (60) par une seconde extrémité libre (67) à l'état expansé de l'extenseur (60).

17. Extenseur selon l'une des revendications 1 à 16, caractérisé en ce que les fils de filaments formant le treillis (52 ; 62 ; 82 ; 92) sont à des distances variables les uns des autres et/ou comportent un diamètre variable.

18. Extenseur selon l'une des revendications 1 à 17, caractérisé en ce que le treillis (82 ; 92) est formé sur une forme présentant un contour variable en direction axiale et/ou radiale.

19. Extenseur selon l'une des revendications 1 à 17, caractérisé en ce que la forme du treillis (82 ; 92) est produite par le biais d'une déformation thermique.

20. Extenseur selon l'une des revendications précédentes, caractérisé en ce que l'extenseur (1) est agencé de telle manière qu'il peut être introduit dans un corps creux au moyen d'un dispositif d'application (10).

21. Extenseur selon la revendication 20, caractérisé en ce que le dispositif d'application (10) comporte un dispositif de réception et de déplacement (11), une gaine d'application (15) et un tampon conique (16).

22. Extenseur selon la revendication 21, caractérisé en ce que le dispositif de réception et de déplacement (11) comporte une extrémité (12) qui s'épanouit sensiblement en cône.

23. Extenseur selon la revendication 21 ou 22, caractérisé en ce que le dispositif de réception et de déplacement (11) est agencé et dimensionné de telle manière qu'il peut être inséré dans la lumière (13) de la gaine d'application (15).

24. Extenseur selon l'une des revendications 21 à 23, caractérisé en ce que le dispositif de réception et de déplacement (11) comporte une lumière (14).
